(19) Europäisches Patentamt European Patent Office Office européen des brevets

(11) **EP 3 698 715 A1**

(12) **EUROPEAN PATENT APPLICATION**

(43) Date of publication:
**26.08.2020 Bulletin 2020/35**

(51) Int Cl.:
***A61B 5/11*** *(2006.01)*

(21) Application number: **19158024.0**

(22) Date of filing: **19.02.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **FERREIRA DOS SANTOS DA FONSECA, Pedro Miguel**
  **5656 AE Eindhoven (NL)**
• **GRASSI, Angela**
  **5656 AE Eindhoven (NL)**

• **VAN DER SANDEN, Henricus Theodorus Johannus Antonius Gerardus**
  **5656 AE Eindhoven (NL)**
• **KRAGT, Rene Dick**
  **5656 AE Eindhoven (NL)**
• **AGAFONOV, Aleksei**
  **5656 AE Eindhoven (NL)**
• **GELISSEN, Jozef Hubertus**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **A SLEEP MONITORING AND POSITION THERAPY SYSTEM AND METHOD**

(57) A sleep monitoring and position therapy system uses signals generated by a movement sensing arrangement to determine a sleep position, identify sleep-disordered breathing events and provide an indication of the level or impact of sleep-disordered breathing for each encountered sleep position. In this way, the invention provides information about disordered breathing associated with different sleep positions, so that a position therapy system may provide improved results.

FIG. 2

EP 3 698 715 A1

## Description

FIELD OF THE INVENTION

This invention relates to sleep monitoring during position therapy.

BACKGROUND OF THE INVENTION

Sleep-disordered breathing (SDB) is an umbrella term for several chronic conditions in which partial or complete cessation of breathing occurs many times throughout the night.

The most common SDB is obstructive sleep apnea (OSA) (or obstructive sleep apnea syndrome (OSAS)) where collapses of the airway cause an obstruction and a consequent reduction (hypopnea) or cessation (apnea) of airflow with continued respiratory effort. This leads to a decrease in blood oxygen saturation, resulting in a cortical arousal and a sudden burst in sympathetic activity with an accompanying increase in heart rate and blood pressure.

Repetitive apnea and hypopnea events cause brief awakenings from sleep, leading to sleep fragmentation and excessive daytime sleepiness.

These respiratory events are defined by the AASM (American Academy of Sleep Medicine).

Apnea is defined as a drop in peak signal excursion by $\geq$ 90% of the pre-event baseline for $\geq$ 10 seconds, wherein the signal is an oronasal thermal signal, PAP device flow signal, or an alternative apnea sensor signal.

An apnea event is scored as:

 obstructive, if it is associated with continued or increased inspiratory effort throughout the entire period of absent airflow;
 central, if it is associated with absent inspiratory effort throughout the entire period of the absent airflow; and
 mixed if it is associated with absent inspiratory effort in the initial portion of the event, followed by resumption of inspiratory effort in the second portion of the event.

There are currently two hypopnea definitions, "recommended" and "acceptable"). A hypopnea event is categorized as:

 recommended if there is a drop in peak signal excursion by $\geq$ 30% of the pre-event baseline for $\geq$ 10 seconds, wherein the signal is the nasal pressure, a PAP device flow signal, or an alternative hypopnea sensor signal, and there is a $\geq$ 3% oxygen desaturation from the pre-event baseline OR the event is associated with a cortical arousal measured on EEG; and
 acceptable if there is a drop in peak signal excursion

by $\geq$ 30% of the pre-event baseline for $\geq$ 10 seconds (again using nasal pressure, PAP device flow, or an alternative hypopnea sensor). There is a $\geq$ 4% oxygen desaturation from the pre-event baseline.

The apnea-hypopnea index (AHI) is defined as the average number of apneas and hypopneas per hour of sleep and it is used to determine the OSAS severity (AHI<5: Normal, AHI 5-15: Mild, AHI 15-30: Moderate, AHI $\geq$ 30: Severe).

The gold standard for the diagnosis of SBD is overnight polysomnography (PSG).

A PSG system monitors many body functions, including brain activity with electroencephalography (EEG), eye movements with electrooculography (EOG), muscle activity or skeletal muscle activation with electromyography (EMG), and heart rhythm with electrocardiography (ECG), during sleep. In a clinical context, PSG commonly includes additional sensors to measure breathing, such as oronasal airflow, surrogate measures of respiratory effort with respiratory inductance plethysmography (RIP) or piezoelectric belts around the thorax and abdomen and peripheral pulse oximetry. Although this technique provides accurate results and is the gold standard technique for the diagnosis of sleep disorders and assessment of sleep, several disadvantage are also present, such as the high complexity, high costs and the burden for the patient.

Alternative portable diagnostic systems have been developed and are often used as home sleep tests (HSTs) for the diagnosis of SDB. They are simpler than PSG, and comprise a smaller set of sensors, are more comfortable for the patient and usually employ automatic algorithms to detect OSAS. These devices record at least three channels of data: airflow, respiratory effort and oxygen saturation.

These HST devices are appropriate for the detection of SDB, however, they are also not comfortable for prolonged, unobtrusive monitoring.

Alternative less obtrusive devices capable of evaluating OSAS have been explored, mainly based on oximetry, respiration analysis, ECG, sounds, or combined approaches. These alternative techniques are based on the observation that during OSA subjects also show an alteration in the heart rate variability (HRV). Several consumer sleep trackers, based on photoplethysmography (PPG), ballistocardiography (BCG), or Doppler radar, have been explored to monitor cardiorespiratory activity during the night.

Clinical research has shown that many OSAS patients experience increased airway obstruction during sleep when lying on their backs compared to when lying on their sides. When an individual experiences an increased level of airway obstruction when lying on their back as compared to any other position, this condition is known as Positional OSAS (POSAS). POSAS is defined as OSAS whose AHI level is at least twice as high when lying on the back (supine position) than in any other position (AHI supine > 2 x AHI other positions).

Treatments like Continuous Positive Airway Pressure (CPAP) and oral appliances can be effective for POSAS patients when used properly. However, a variety of issues may limit one's ability to use these devices regularly.

An alternative treatment is positional therapy (PT). This is a behavioral strategy to treat POSAS. In recent years, many PT strategies have been designed to prevent patients from sleeping on their back, such as an alarm system, a backpack with ball, behavioral therapy, a pillow with straps, and the so-called tennis ball technique (by which a physical device is used to avoid the supine position). Another option is the use of a small device that uses "vibro-tactile feedback" technology. Worn on the back of the neck or in a belt around the chest, it gently vibrates when the patient start to sleep on the back. Without waking the patient up, the vibration alerts the body to a change in position. PT can be used alone or together with another sleep apnea treatment.

It would be desirable to measure the AHI (apnea/hypopnea events) during PT in order to assess the POSAS severity and the effectiveness of the therapy. In addition, showing the AHI values as a feedback to the patients could motivate them thereby increasing the adherence to the therapy. PSG is not applicable for long-term therapy such as PT, since it is obtrusive, expensive and it requires a sleep laboratory and a sleep technician. HSTs (with a reduced polygraphic setup) are portable, but still rely on sensing methods that are uncomfortable and impractical for prolonged usage.

Alternative sensor measurements have been explored, mainly based ECG, oximetry, respiration analysis, sounds, or combined approaches. Using ECG, it is possible to measure the alteration in the HRV due to sleep breathing events, however it is not practical for long-term use. It introduces additional requirements for the consumer solution, such as skin contact, that can clearly reduce the comfort of the device and, consequently the patients' compliance to the PT.

There is therefore a need for a system which allows monitoring of breathing events during position therapy in a less obstructive and low cost manner.

## SUMMARY OF THE INVENTION

The invention is defined by the claims.

According to examples in accordance with an aspect of the invention, there is provided a sleep monitoring and position therapy system, for monitoring a subject, comprising:

> a movement sensing arrangement; and
> a controller, which is adapted during a sleep monitoring period to:

>> determine from the movement sensing arrangement output signals a sleep position out of a set of possible sleep positions;
>> identify from the movement sensing arrangement output signals sleep-disordered breathing events; and
>> provide an indication of the level or impact of sleep-disordered breathing for each encountered sleep position.

This system monitors subject movements both to determine their sleep position, for use in controlling a position therapy system, and also detects sleep-disordered breathing from the movement signals. The movement signals for example relate to measurement of seismocardiographic and/or ballistocardiographic forces resulting from cardiac activity. The use of movement sensing is unobtrusive and can easily be integrated into a wearable device for PT for OSAS patients.

The system enables the level or impact of sleep-disordered breathing to be determined for different sleep positions, and this can be used to improve a position therapy approach.

The sleep-disordered breath events for example comprise sleep apnea events. As discussed above, they may comprise apnea or hypopnea events, both of which are considered to fall within the general term "apnea".

In one example, the movement sensing arrangement comprises an acceleration sensor or gyroscope sensor arrangement for recording ballistocardiography signals, and the controller is adapted to determine the sleep-disordered breathing events based on analysis of the ballistocardiography signals.

A ballistocardiography (BCG) system for example monitors movement/force signals in the longitudinal direction along the aorta, hence in the head-foot direction of the subject. The controller may then be adapted to determine the timing of each heart beat from the movement sensing arrangement signals and determine the sleep-disordered breath events from an inter-beat interval time series. In this way, features of the heart beats are identified from the BCG signals.

In another example, the movement sensing arrangement comprises an acceleration sensor or gyroscope sensor arrangement for recording seismocardiography signals, and the controller is adapted to determine the sleep-disordered breathing events based on analysis of the seismocardiography signals.

A seismocardiography (SCG) system for example monitors movement/force signals at the surface of the chest, for example in front-back direction of the subject. The controller may then be adapted to determine repetitive patterns in the movement sensing arrangement signals to determine an inter-beat interval time series and determine the sleep-disordered breath events from the inter-beat interval time series. In this way, the SCG signals are not used to identify specific portions of the heart beat signal, but time periods are examined in a more abstract way.

In both cases, the inter-beat interval time series may be provided to a machine learning classifier to identify the sleep-disordered breath events. The movement sensing arrangement may comprise a tri-axial acceleration sensor arrangement.

The level of sleep-disordered breathing for each encountered sleep position may comprise an apnea-hypopnea index value. However, other measures of the level of sleep-disordered breathing may be used. The measure may for example comprise an indication of the impact of those events on the quality of sleep.

The system may further comprise one or more of:

> a sensor arrangement for detecting respiratory effort;
> a microphone for detecting breathing sounds.

Respiratory information provides additional input to assist in determining the breathing events. The controller may for example be adapted to detect snoring from the breathing sounds. Positional therapy is known to relieve snoring and a measure of snoring thus provides additional feedback information.

The controller may be adapted to determine sleep stages, and thereby determine a sleep time period and an awake time period during the monitoring period. In this way, sleep disordered breathing events may be categorized as taking place during sleep or discounted if during wakefulness. In this way, a better measure of the level of breathing disorder during sleep is possible.

The controller may be adapted to extract from the movement sensing arrangement output signals separate signal components, for detection of sleep position, respiratory movements and seismocardiography or ballistocardiography signals. Thus, the same movement signals may be processed by different signal processing paths to provide optimum signal processing.

The system preferably further comprises a position therapy device for providing a stimulus to induce the subject to change sleep position. This stimulus may take various forms as discussed above.

The invention also provides a sleep monitoring method for monitoring a subject, comprising:

> monitoring movements of the subject;
> determining from the monitored movements a sleep position out of a set of possible sleep positions;
> identifying from the movement sensing arrangement output signals sleep-disordered breathing events; and
> providing an indication of the level or impact of sleep-disordered breathing for each encountered sleep position.

The method may further comprise providing a stimulus to induce the subject to change sleep position.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Figure 1 shows a sleep monitoring and position therapy system;
> Figure 2 shows the signals of interest for ballistocardiography;
> Figure 3 shows the signals of interest for a seismocardiography;
> Figure 4 shows how SCG or BCG can be used to provide an indication of the level or impact of sleep-disordered breathing;
> Figure 5 shows a modification to Figure 4 which further takes account of respiration;
> Figure 6 shows the method carried out by the overall system of Figure 1;
> Figure 7 shows the signals from the three axes of a DC-response tri-axial accelerometer, when there are changes in body position;
> Figure 8 shows the signals from the three axes measured with a gyroscope for the same period as Figure 7; and
> Figure 9 shows a modification to Figure 6 in which one or more microphones are used to measure breathing sounds.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sleep monitoring and position therapy system in which signals generated by a movement sensing arrangement are used to determine a sleep position, identify sleep-disordered breathing events and provide an indication of the level or impact of sleep-disordered breathing for each encountered sleep position. In this way, the invention provides information about disordered breathing associated with different sleep positions, so that a position therapy system may provide improved results.

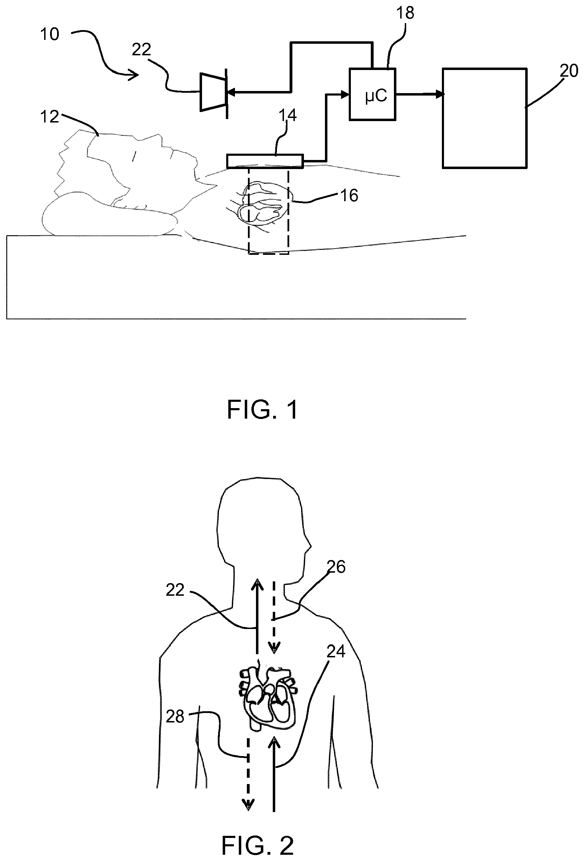

Figure 1 shows a sleep monitoring and position therapy system 10, for monitoring a subject 12. The system comprises a movement sensing arrangement 14 for monitoring movements induced by the beating of the heart and movements of the subject, for example between different sleeping positions. The movement sensing arrangement for example comprises an acceleration sensor such as an accelerometer or gyroscope sensor, mounted on a belt which positions the sensor on the chest of the subject over the heart 16. The movement sensing arrangement is thus positioned close to the location of the heart and is also preferably fixed to the surface of the chest, e.g. with an adhesive in contact with the skin, or by virtue of the integration in a stretchable belt. It may instead be integrated in a garment worn by the subject.

The movement sensing arrangement output signals (which will be termed "movement signals" below) are provided to a controller 18. The controller performs analysis of the movement signals to determine a sleep position out of a set of possible sleep positions and also to identify sleep-disordered breathing events.

The sleep positions for example include supine (facing upwards), prone (facing downwards) or on the left side or on the right side. These positions may be determined based on the position of the torso, or the neck/head position using a sensor mounted on the neck. More complex body positions could be detected, including for example when the head orientation differs from the torso orientation, which can also have an impact on the sleep apnea or the severity of sleep apnea. A combination of multiple sensors could for example be used for this purpose mounted on different parts of the body and/or or on or under the mattress.

In combination, this information is used to provide an indication of the level or impact of sleep-disordered breathing for each encountered sleep position. For this purpose, an output device 20 is shown. The system is also for providing position therapy, and for this purpose a position therapy device 22 is provided for generating a stimulus to induce the subject to change sleep position. This is shown as a speaker in Figure 1, but any known position therapy device may be used as discussed above.

As discussed further below, the movement sensing arrangement 14 may include other sensing modalities, such as a sensor arrangement for detecting respiratory effort and a microphone for detecting breathing sounds.

The invention implements detection of breathing events based on movement signals. There are basically two different types of movement signal which may be used, described below.

Ballistocardiography (BCG) is a well-known method for measuring certain aspects of cardiac activity.

Figure 2 shows the signals of interest. When the heart ejects blood (arrow 22), it does so mainly through the ascending aorta, in a longitudinal, upward direction. When the heart pulls blood from the body, it does so also in a longitudinal direction, parallel to the spine (arrow 24). These longitudinal forces exerted by the heart on the blood are, according to Newton's third law, matched by equal forces by the blood on the body, with opposite directions (arrows 26 and 28).

$$\vec{F}_{blood} = -\vec{F}_{body}$$

By means of an accelerometer, the accelerations on the body caused by these forces are measured. According to Newton's second law, and since the mass can be considered constant during a small interval of a ballistocardiographic measurement, acceleration is directly related to the force exerted on a certain mass through the equation:

$$F = m \cdot a$$

It follows immediately that the acceleration measured on a body resting on a moving surface is related to the acceleration of the blood,

$$a_{blood} = \frac{-m_{body+surface} \cdot a_{body+surface}}{m_{blood}}$$

Since the mass of the body, surface and blood will stay approximately constant during a measurement, it follows that:

$$a_{blood} = a_{body+surface} \cdot c$$

where c is a negative constant.

Early ballistocardiograms were recorded with rather complicated apparatus, whereby a patient was laid down on either a suspended surface or on a surface with very low friction and a sensor would then measure longitudinal displacement, velocity or acceleration of that surface. Developments in digital electronics, signal processing and sensor technology have enabled more convenient and simpler set-ups. Since then, it has been shown that the ballistocardiogram can also be measured under the subject's body with sensors such as strain gauges, pressures sensors and load cells under the feet of a bed.

The example of Figure 1 is based on an accelerometer mounted on the chest of the subject and set up to measure longitudinal accelerations. This can be used to measure the acceleration of the blood caused by cardiac activity.

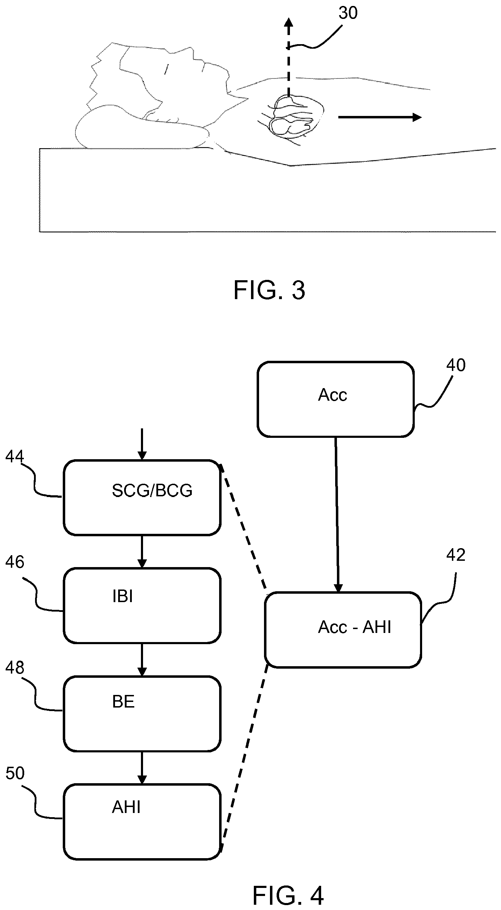

While BCG measures the ballistic effect of the beating heart, mainly in a direction approximately coinciding with a longitudinal direction, seismocardiography (SCG) measures the small vibrations produced on the surface of the chest by the heart contractions and blood ejection. These vibrations are mostly measurable in a direction orthogonal to the surface of the chest. This is shown as arrow 30 in Figure 3 and can be picked up, for example, by sensitive accelerometers configured to respond to orthogonal accelerations when mounted on the chest, close to the approximate location of the heart.

Figure 4 shows how SCG or BCG signals can be used to provide an indication of the level or impact of sleep-disordered breathing.

The movement sensing arrangement provides movement signals, in particular acceleration signals, in step 40. This is achieved with a sensor which measures seismocardiographic and/or ballistocardiographic forces resulting from cardiac activity. An accelerometer or a gyroscope is configured to respond to forces and/or accelerations orthogonal to the surface of the chest wall for SCG or longitudinal forces, along the direction of the body (or the bed) for BCG.

The movement signals are converted to a measure of the degree of sleep-disordered breathing in step 42. In this example, the AHI measure described above is obtained.

Step 42 comprises the sub-steps of obtaining the BCG and/or SCG signals in step 44, and determining the inter-beat intervals (IBI) in step 46. This for example involves detecting the time location of each individual heart beat, and computing a time series based on the distance between consecutive heart beats.

It is noted that the processing to obtain the IBI is different for BCG and SCG signals. Although seismocardiography (SCG) is, to a certain extent, related to ballistocardiography (BCG) insofar as both measure the mechanical (as opposed to electrical) activity of the heart, the determination of the IBI for an SCG is less straightforward, since the signal is far more complex than a BCG signal. Thus, different algorithms are needed.

One approach is to find repetitive patterns in the SCG signal which have an expected length (determined by the shortest and longest duration between heart beats) without relying on the presence of peaks per se, as is used in the analysis of BCG (and ECG) signals. In particular, analysis of a BCG signal involves identifying the presence of components of the signal which are related to specific events of the cardiac cycle (e.g. the J-peak in the BCG). The SCG signal has a more complex and variable shape, so instead of relying on the identification of a specific peak or component of the signal, the distance between these repetitive patterns is identified in order to determine the inter-beat interval. Thus, the step of detecting the precise location of heart beats in the signal based on specific elements of the signal is not carried out (as for BCG). From the repetitive occurrence of a pattern, the inter-beat intervals can be computed without identifying specific elements of that pattern.

In step 48, there is the detection of breathing events (BE). The inter-beat interval (IBI) time series is used as an input to a pre-trained machine learning model, by which the system automatically detects the presence of breathing events, such as obstructive apneas, central apneas, and hypopneas.

The detection of breathing events can be performed using any or a combination of known methods which are based on ECG signals. Although the sensing modality is fundamentally different (electrical activity for ECG, in contrast with mechanical activity for SCG/BCG), the resulting inter-beat intervals are comparable and as such, known methods can be applied.

These can be divided in two main categories, which can be used alternatively, or in combination:

A first approach is to use the IBI series directly as the input to a machine learning classifier. In this case the classifier is trained to recognize certain patterns directly of the IBI time series and use those patterns to detect the presence of breathing events. Modern machine deep learning methods make this a viable option, with the advantage of requiring less development time and less domain knowledge to achieve the end goal. They have the disadvantage of requiring a large (and representative) enough training data set.

A second approach is to use the IBI series to derive a number of intermediate, manually engineered parameters which are known to describe different patterns and behaviors of IBIs during and following breathing events. These features, commonly describing heart rate variability (HRV) parameters, can be based on the time and frequency analysis of the IBI series and have been described in literature to be successful for this task. This approach has the advantage of using domain knowledge to reduce the complexity and training requirements of the machine learning classifier, often requiring less data to achieve a good performance.

In step 50 the AHI is calculated, reflecting the average number of breathing events per hour.

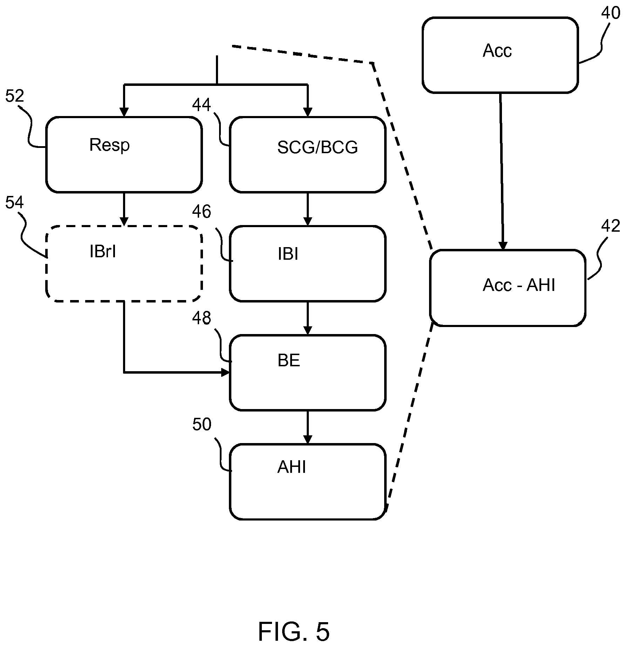

Figure 5 shows a modification to Figure 4 which further takes account of respiration.

The derivation of the AHI from the movement signals further comprises the sub-step of obtaining a respiration signal in step 52.

Respiratory effort may be measured with sensors placed on the subject's body. The air inhaled via the nose or the mouth causes the lungs to inflate and, consequently, the chest circumference to expand. Because the volume of the chest will increase, it will exert a downwards force on whatever surface the subject is lying on and an upward force on whatever sensor might be mounted on the chest of the subject. Thus, respiration will also generate a force as shown by arrow 30 in Figure 3, but with a different frequency spectrum to the forces created by the heart beat. A movement sensing arrangement configured to respond to both orthogonal and well as longitudinal forces, placed on the chest of the subject, close to the heart, can be therefore be used simultaneously to measure BCG signals as well as respiratory activity. The separation of the BCG signals from the respiratory activity signals is then simply based on the direction of sensing.

Orthogonal forces alone may be used to measure simultaneously SCG signals as well as respiratory activity. The separation of the SCG signals from the respiratory activity signals is then based on a spectral analysis.

As shown in Figure 5, the respiratory movement signals are used as an additional input for the detection of breathing events in step 48. This can be done with the "raw" respiratory movement signal (i.e. without the optional step 54), in which case the machine learning (deep) model can learn the patterns in the respiratory movements that are associated with the presence of breathing events, or based on defined features describing time (amplitude, volume, depth, etc.) or frequency (rate, regularity, etc.) characteristics of the signal.

The optional step 54 in Figure 5 is to determine inter-breath intervals (IBrI).

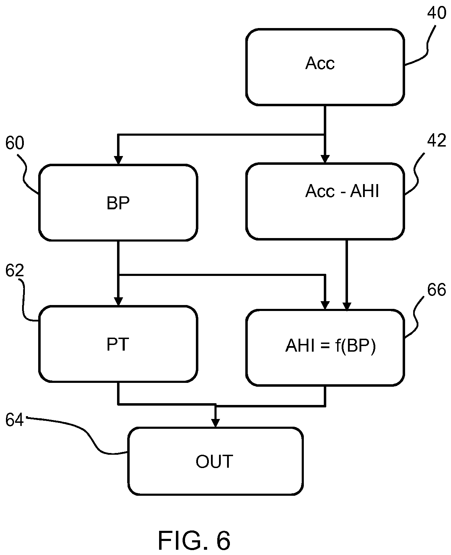

Figure 5 shows the separation of the different components of the movement signal into two branches prior to the estimation of AHI. This serves more than the mere purpose of filtering the BCG/SCG signal to be able to detect inter-beat intervals. A further component is also used for position sensing (as shown in Figure 6, described below).

Thus, the movement signal will have different components in different directions, and the processing of different components is able to separate SCG signals from BCG signals. Different components are also caused by the influence of different physiological mechanisms on the signal, such as the influence of body position, where the gravitational component has an influence on signals of all acceleration directions not orthogonal to the direction of gravity, and the influence of breathing and chest movements which are most visible on signals orthogonal to the surface of the torso/chest. These components can be identified and separated in different ways. For example, a tri-axial accelerometer will respond to displacements and accelerations in well-defined directions. The alignment of the accelerometer can be such that one axis is aligned in a direction orthogonal to the surface of the chest, and hence also along the direction of gravity when lying in a supine or prone position. Another axis could be aligned along the gravity direction when lying on the side. The third axis could be mounted in a direction perfectly orthogonal to gravity when in those two positions, responding only to displacements/accelerations in the longitudinal direction. In this case, the first axis would have components of SCG, breathing and gravity (when lying on supine or prone positions), the second would have components of gravity when lying on the side, and the third would have components of BCG.

The different physiological components in the signal may also be separated using different band-pass filtering strategies (configured to respond to expected signal frequency bands for each physiological component), time-frequency analysis techniques such as wavelet decompositions of the accelerometer signal(s), or other non-linear techniques such as empirical mode decomposition.

The aim of these techniques is to separate the different physiological processes (body position and body movements, breathing, and cardiac activity) in the signals for further (separate) processing.

Figure 6 shows the method carried out by the overall system.

The determination of AHI is carried out in steps 40 and 42 as explained above with reference to Figures 4 and 5.

In addition, the movement signals are used to determine the body position (BP) of the subject in step 60. This can be achieved when tri-axial accelerometers are used, so that the lying position of the subject can be identified. This body position is used to implement position therapy (PT) in step 62. A position therapy system may be passive (by providing a mechanism to prevent a subject sleeping in a supine position) or active. An active approach involves applying a stimulus to the subject to induce them to move position when they are in a supine position.

As mentioned above, this active stimulus may involve an alarm system or vibro-tactile feedback technology. Sensors used for the position therapy may be at additional body locations (not only on the chest) such as for measuring head position (with a tri-axial accelerometer mounted on the head (e.g. on a head-band, or on another face-mounted device such as a CPAP mask), or even mounted outside the body, for example on a sensor mattress which is able to measure the body position.

An output is provided in step 64 to users (i.e. the subject) and/or clinicians in order to give feedback about the effectiveness of the position therapy.

Furthermore, the output provides an indication of the level or impact of sleep-disordered breathing for each encountered sleep position. This takes place in step 66, wherein the AHI value obtained in step 42 is processed using the body position information from step 60. In the example of Figure 6, this is represented as an AHI value for each body position, i.e. an AHI value which is a function of body position (AHI = f(BP)). In other words, position-specific AHI values are determined.

In standard position therapy PT, after each night or after a number of nights, the subject and/or the referring clinician can have information about the percentage of time the subject was lying in a supine position, and the percentage of time lying sideways; however, this information might not be sufficient to assess the efficacy of the therapy. The system of Figure 6 enables the information to be complemented with information about the overall AHI (which should reflect residual - i.e. untreated apneas/hypopneas) and position-specific AHI, which should highlight whether and how position therapy is helping prevent additional breathing events.

Different sensor options are possible to measure SCG and/or BCG. These sensors can consist of a selected one of different sensor types, or a combination of different types of sensors, including accelerometers (AC- or DC-response) or gyroscopes. As is clear from the discussion above, they may respond to the forces and accelerations in a direction orthogonal to the chest wall and/or the forces and accelerations in the longitudinal direction (along the direction of the body/bed).

The same sensor used for SCG/BCG measurements may be used to measure body position, needed for position therapy, and for this purpose a multi-axis (preferably tri-axial) DC-response accelerometer is preferable.

AC-response accelerometers, even when mounted on the body, cannot measure static accelerations such as those related to gravity. This makes them unsuitable for measuring body position. If AC-response accelerometers are desired, the system may comprise at least two accelerometers, one configured to measure body position (a DC-response accelerometer) and another (which may then be an AC-response accelerometer) configured to measure cardiac and respiratory information. The same is true when using gyroscopes instead of accelerometers. Gyroscopes may be used for SCG and BCG measurements. Gyroscopes do not respond in the same way to the pull of gravity, and cannot (easily) be used for measuring body position.

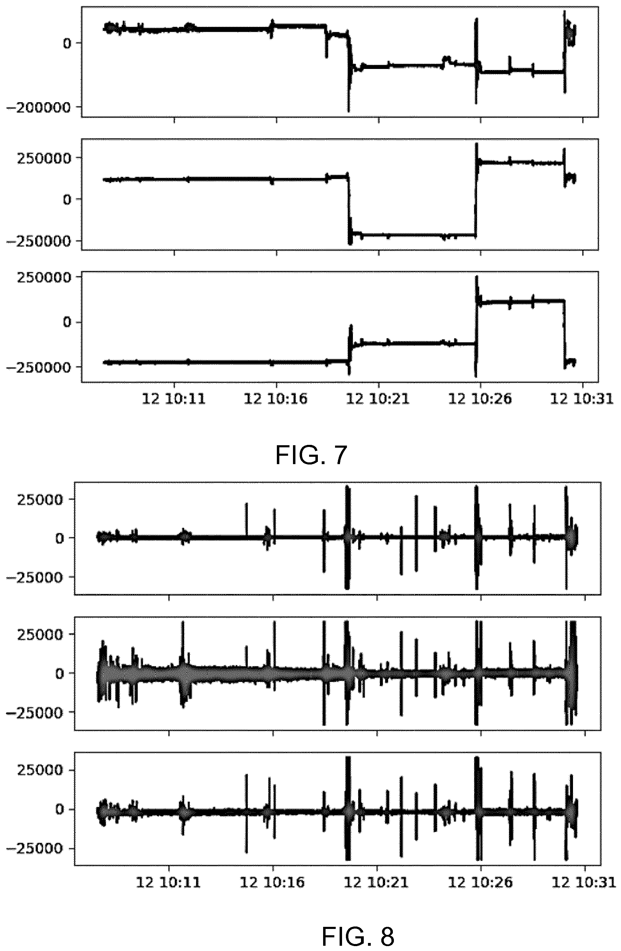

For example, Figure 7 shows signals from the three axes of a DC-response tri-axial accelerometer, where changes in body position are clearly visible as "steps" in the signal.

Figure 8 shows the signals from the three axes measured with a gyroscope for the same period as Figure 7. It is less trivial to measure, on each point in the recording, the body position of the subject. Thus, the system may combine a gyroscope and a DC-response accelerometer mounted on the body.

It is instead possible to use a DC-response accelerometer mounted on the body as a single sensor. However, there are advantages to using an AC-response accelerometer or a gyroscope for the BCG/SCG measurement. By construction, because they do not respond to the component of gravity, they can use the entire dynamic range after analog-to-digital conversion without requiring dedicated filtering before conversion. This means that they can be more sensitive than DC-response accelerometers for a comparable resolution.

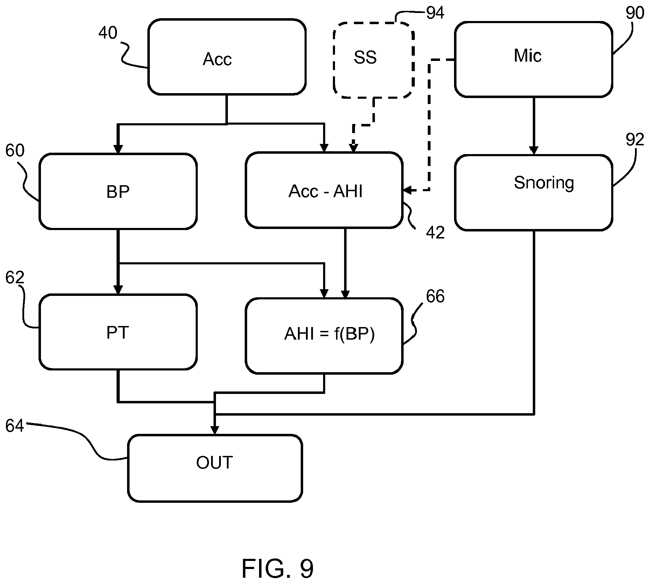

Figure 9 shows a modification to Figure 6 in which one or more microphones are used, in step 90, to measure breathing sounds. The microphone or microphones are preferably mounted as part of the movement sensing arrangement, but alternatively they can be contactless, for example mounted on the night table next to the sleeping subject, as a separate device connected to the system, or alternatively using a device with a microphone that the user might have (e.g. smartphone). The microphone or microphones are used to measure breathing sounds, in particular those associated with relevant breathing events that are known to be associated with OSAS, such as snoring, gasping, etc.

Figure 9 also shows a step 92 of detecting snoring from the breathing sounds. Since positional therapy is known also to relieve snoring, this information can be further provided to the user and/or clinician, highlighting the efficacy of the therapy also to this (secondary) aspect.

The presence of these breathing sounds can also be used to further improve the detection of breathing events. This is shown in Figure 9 by the dotted arrow from step 90 to step 42.

A further option is to use cardiac and respiratory information to automatically detect sleeping and wakening periods, and/or sleep stages. This is shown as step 94 in Figure 9. This can be performed using many different known methods, for example as described in P. Fonseca, N. den Teuling, X. Long, and R. M. Aarts, 'Cardiorespiratory sleep stage detection using conditional random fields,' IEEE Journal of Biomedical and Health Informatics, vol. 21, no. 4, pp. 956-66, 2017.

For example, heart rate variability (HRV) may be computed from an ECG signal, but equally from the heart beats detected using the SCG or BCG signals, and respiratory variability (RV) for example derived from an accelerometer mounted on the torso. Based on HRV and RV characteristics that are known to be discriminative of different sleep stages, a pre-trained machine learning classifier may be used to predict, e.g. for each segment (e.g. of 30 seconds) in a recording, the sleep state (awake vs. asleep, or even awake vs. REM vs. N1 vs. N2).

Body movements may also be used to discriminate between awake and sleeping states.

This information can in turn be used to compute the total sleep time during each recording.

The total sleep time can then be used, together with a calculation of the number of apneas and hypopneas, to compute the sleeping AHI, by dividing the number of detection events (apneas plus hypopneas) by the total sleep time, arriving at the number of events per hour of sleep.

This estimation is more accurate than the estimation based on total recorded AHI (number of events per hour of recording). The difference in accuracy is particularly visible for subjects that spend a large part of the night awake, thereby reducing the recorded AHI value, and hence giving an underestimated view on the severity of their condition.

The detected sleep stages can also be used to provide additional feedback to users and/or clinicians regarding their sleep architecture and overall quality of sleep.

The invention makes use of motion sensing for both cardiac monitoring and position detection. For example, heart beats are measured unobtrusively and possibly without direct skin contact, and potentially embodied in the same module as used for PT.

The SCG and BCG signals are obtained based on a sensor mounted against the subject in the examples above. However, these signals can also be measured with bed sensors, mounted on or below the mattress. This has the disadvantage of requiring additional elements separate from the main PT device.

The examples above derive an AHI value. However, instead of calculating an estimate of AHI, a similar algorithm could be configured to provide an alternative measure that reflects the impact of apneas on the overall quality of sleep; such a measure could combine a quantification or qualification of one or more of the following elements:

> a measure of the intensity and/or frequency of disordered breathing events;
> a measure of the effect of breathing events on the architecture of sleep, for example, on the number of awakenings, the number of transitions between sleep stages, and/or percentage of sleep stages known to be affected by the presence of disordered breathing, such as N3 (also known as slow wave sleep or deep sleep) or REM sleep; and
> a measure of the number of (autonomic) arousals following disordered breathing events.

Although these alternative measures would not have the same meaning and definition as AHI, it should be clear that they also reflect the effect of sleep apnea on the quality of sleep, and in turn, the improved effect arising from the use of PT.

Thus, the information of relevance to the subject or clinician is the level or impact of sleep-disordered breathing. This is derived for each encountered sleep position (i.e. each sleep position which the subject adopted during the sleep monitoring period). In this way, the position-specific impact/level is obtained as explained above.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. The computer program discussed above may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A sleep monitoring and position therapy system, for monitoring a subject, comprising:

> a movement sensing arrangement (14); and
> a controller (28), which is adapted during a sleep monitoring period to:

>> determine from the movement sensing arrangement output signals a sleep position out of a set of possible sleep positions;
>> identify from the movement sensing arrangement output signals sleep-disordered breathing events; and
>> provide an indication of the level or impact of sleep-disordered breathing for each encountered sleep position.

2. A system as claimed in claim 1, wherein the sleep-disordered breath events comprise sleep apnea events.

**3.** A system as claimed in claim 1 or 2, wherein the movement sensing arrangement (14) comprises an acceleration or gyroscope sensor arrangement for recording ballistocardiography signals, and the controller is adapted to determine the sleep-disordered breathing events based on analysis of the ballistocardiography signals.

**4.** A system as claimed in claim 3, wherein the controller (18) is adapted to determine the timing of each heart beat from the movement sensing arrangement signals and determine the sleep-disordered breath events from an inter-beat interval time series.

**5.** A system as claimed in claim 1 or 2, wherein the movement sensing arrangement (14) comprises an acceleration or gyroscope sensor arrangement for recording seismocardiography signals, and the controller is adapted to determine the sleep-disordered breathing events based on analysis of the seismocardiography signals.

**6.** A system as claimed in claim 5, wherein the controller (18) is adapted to determine repetitive patterns in the movement sensing arrangement signals to determine an inter-beat interval time series and determine the sleep-disordered breath events from the inter-beat interval time series.

**7.** A system as claimed in any one of claims 1 to 6, wherein the level of sleep-disordered breathing for each encountered sleep position comprises an apnea-hypopnea index value.

**8.** A system as claimed in any one of claims 1 to 7, further comprising one or more of:

> a sensor arrangement for detecting respiratory effort;
> a microphone for detecting breathing sounds.

**9.** A system as claimed in any one of claims 1 to 8, wherein the controller (18) is adapted to detect snoring from the breathing sounds.

**10.** A system as claimed in any one of claims 1 to 9, wherein the controller (18) is adapted to determine sleep stages, and thereby determine a sleep time period and an awake time period during the monitoring period.

**11.** A system as claimed in any one of claims 1 to 10, wherein the controller (18) is adapted to extract from the movement sensing arrangement output signals separate signal components, for detection of sleep position, respiratory movements and seismocardiography or ballistocardiography signals.

**12.** A system as claimed in any one of claims 1 to 11, further comprising a position therapy device for providing a stimulus to induce the subject to change sleep position.

**13.** A sleep monitoring method for monitoring a subject, comprising:

> (40) monitoring movements of the subject;
> (60) determining from the monitored movements a sleep position out of a set of possible sleep positions;
> (42) identifying from the movement sensing arrangement output signals sleep-disordered breathing events; and
> (64, 66) providing an indication of the level or impact of sleep-disordered breathing for each encountered sleep position.

**14.** A method as claimed in claim 13, further comprising (62) providing a stimulus to induce the subject to change sleep position.

**15.** A computer program comprising computer program code means which is adapted, when said program is run on a computer, to implement the method of claim 13 or 14.

FIG. 1

FIG. 2

FIG. 3

FIG. 4

FIG. 5

FIG. 6

FIG. 7

FIG. 8

FIG. 9

Europäisches
Patentamt

European
Patent Office

Office européen
des brevets

# EUROPEAN SEARCH REPORT

Application Number

EP 19 15 8024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/201419 A1 (HANCOCK MEDICAL INC [US]) 23 November 2017 (2017-11-23) | 1,2, 7-11,13, 15 | INV. A61B5/11 |
| Y | * paragraphs [0046] - [0078], [0111] - [0112], [0136]; figures 1,2,7,15 * | 3-6,12, 14 | |
| Y | US 2017/042471 A1 (MERIHEINÄ ULF [FI]) 16 February 2017 (2017-02-16) * the whole document * | 3-6 | |
| A | WO 2018/081778 A1 (MC10 INC [US]) 3 May 2018 (2018-05-03) * paragraphs [0037] - [0040] * | 1,3,5,12 | |
| Y | US 2015/173672 A1 (GOLDSTEIN DAVID BRIAN [US]) 25 June 2015 (2015-06-25) * paragraphs [0087] - [0088], [0106], [0261] - [0267], [0278] - [0291] * | 12,14 | |
| A | US 2016/354603 A1 (KEENAN DESMOND B [US] ET AL) 8 December 2016 (2016-12-08) * paragraphs [0022] - [0037], [0057]; figures 1-3 * | 1,13 | TECHNICAL FIELDS SEARCHED (IPC) A61B |
| A | US 2010/030085 A1 (ROJAS OJEDA JUAN LUIS [ES] ET AL) 4 February 2010 (2010-02-04) * the whole document * | 11 | |

The present search report has been drawn up for all claims

| Place of search | Date of completion of the search | Examiner |
|---|---|---|
| Berlin | 30 July 2019 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 15 8024

This annex lists the patent family members relating to the patent documents cited in  the above-mentioned European search report.
The members are as contained in the European Patent Office EDP file on
The European Patent Office is in no way liable for these particulars which are merely given for the purpose of information.

30-07-2019

| Patent document cited in search report | | Publication date | Patent family member(s) | | Publication date |
|---|---|---|---|---|---|
| WO 2017201419 | A1 | 23-11-2017 | CN | 109310348 A | 05-02-2019 |
| | | | EP | 3457926 A1 | 27-03-2019 |
| | | | JP | 2019518520 A | 04-07-2019 |
| | | | US | 2019142625 A1 | 16-05-2019 |
| | | | WO | 2017201419 A1 | 23-11-2017 |
| US 2017042471 | A1 | 16-02-2017 | CN | 107920766 A | 17-04-2018 |
| | | | EP | 3334337 A1 | 20-06-2018 |
| | | | FI | 126600 B | 15-03-2017 |
| | | | JP | 6508417 B2 | 08-05-2019 |
| | | | JP | 2018527980 A | 27-09-2018 |
| | | | US | 2017042471 A1 | 16-02-2017 |
| | | | WO | 2017025861 A1 | 16-02-2017 |
| WO 2018081778 | A1 | 03-05-2018 | NONE | | |
| US 2015173672 | A1 | 25-06-2015 | CA | 3004536 A1 | 12-05-2016 |
| | | | CN | 107205693 A | 26-09-2017 |
| | | | EP | 3215010 A1 | 13-09-2017 |
| | | | US | 2015173672 A1 | 25-06-2015 |
| | | | WO | 2016073965 A1 | 12-05-2016 |
| US 2016354603 | A1 | 08-12-2016 | AU | 2016271082 A1 | 23-11-2017 |
| | | | CA | 2986240 A1 | 08-12-2016 |
| | | | CN | 107614056 A | 19-01-2018 |
| | | | EP | 3302694 A1 | 11-04-2018 |
| | | | US | 2016354602 A1 | 08-12-2016 |
| | | | US | 2016354603 A1 | 08-12-2016 |
| | | | US | 2016354608 A1 | 08-12-2016 |
| | | | WO | 2016195809 A1 | 08-12-2016 |
| US 2010030085 | A1 | 04-02-2010 | EP | 2070471 A1 | 17-06-2009 |
| | | | ES | 2298060 A1 | 01-05-2008 |
| | | | US | 2010030085 A1 | 04-02-2010 |
| | | | WO | 2008037820 A1 | 03-04-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **P. FONSECA ; N. DEN TEULING ; X. LONG ; R. M. AARTS.** Cardiorespiratory sleep stage detection using conditional random fields. *IEEE Journal of Biomedical and Health Informatics,* 2017, vol. 21 (4), 956-66